## Europäisches Patentamt

(19) **European Patent Office**

**Office européen des brevets**

(11) Veröffentlichungsnummer: **0 121 091**
**B1**

(12) **EUROPÄISCHE PATENTSCHRIFT**

(45) Veröffentlichungstag der Patentschrift:
29.10.86

(21) Anmeldenummer: **84102026.6**

(22) Anmeldetag: **27.02.84**

(51) Int. Cl.⁴: **C 08 G 69/48,** C 08 G 63/76,
C 08 G 65/32, A 61 K 7/06,
A 61 K 7/11

(54) Mehrfunktionelle quartäre Ammoniumverbindungen mit bis zu 4 quartären Ammoniumgruppen an den Kettenenden, ihre Herstellung und Verwendung in kosmetischen Zubereitungen, insbesondere Haarbehandlungsmitteln.

(30) Priorität: 03.03.83 DE 3307473

(43) Veröffentlichungstag der Anmeldung:
10.10.84 Patentblatt 84/41

(45) Bekanntmachung des Hinweises auf die Patenterteilung:
29.10.86 Patentblatt 86/44

(84) Benannte Vertragsstaaten:
AT BE CH DE FR GB IT LI NL SE

(56) Entgegenhaltungen:
FR - A - 2 094 645
FR - A - 2 168 994

PATENT ABSTRACTS OF JAPAN; unexamined
applications, Field C, Vol. 4, Nr. 81, 11. Juni 1980 THE
PATENT OFFICE JAPANESE GOVERNMENT Seite 39 C
14

(73) Patentinhaber: **Henkel Kommanditgesellschaft auf
Aktien, Postfach 1100 Henkelstrasse 67,
D-4000 Düsseldorf-Holthausen (DE)**

(72) Erfinder: **Ritter, Wolfgang, Dr., Hochdahler Strasse 22,
D-4010 Hilden (DE)**
Erfinder: **Höffkes, Horst, Dr., Carlo-Schmid-Strasse 113,
D-4000 Düsseldorf-Hellerhof (DE)**
Erfinder: **Giede, Karl, Schlehenweg 12, D-4010 Hilden
(DE)**

**Beschreibung**

Die Erfindung schlägt mehrfunktionelle quartäre Ammoniumverbindungen mit einem Molekulargewicht 500–5000 vor, welche quartäre Ammoniumgruppen an beiden Kettenenden tragen und beschreibt deren Herstellung sowie ihre Verwendung in kosmetischen Zubereitungen, insbesondere in Haarnachbehandlungsmitteln, Fönwellen, Festigern, Haarsprays, aber auch Shampoos, Dauerwellmitteln oder Haarfärbemitteln.

Bei der Behandlung des menschlichen Haares, insbesondere bei der Haarnachbehandlung, wird angestrebt, die Nass- und Trockenkämmbarkeit zu verbessern, statische Aufladungen zu verhindern und angenehme Griffeigenschaften (weichen Griff) zu erzeugen. Zu diesem Zweck ist bereits eine Vielzahl von Wirkstoffen bekannt, die 1, 2 oder mehrere quartäre Ammoniumgruppen enthalten. Die bekannten niedermolekularen quartären Ammoniumverbindungen (1–2 quartäre Ammoniumgruppen, meist kationtensidischer Charakter, Molekulargewicht unter 500) – genannt sei z. B. Cetyltrimethylammoniumchlorid – erbringen, wenn sie z. B. in emulsionsförmigen Haarnachbehandlungsmitteln angeboten werden, die gewünschten Eigenschaften. Nachteilig ist jedoch, dass das Haar durch die mehr oder weniger starke Belastung mit Fettstoffen meist den Frisurenhalt schnell verliert. Darüber hinaus können bei dieser Substanzklasse Hautirritationen und Schleimhautreizungen auftreten.

Ausserdem sind sie mit anionischen Tensiden in der Regel nicht verträglich, so dass antistatisch wirksame Shampoos unter gleichzeitiger Verwendung anionischer und kationischer Tenside äusserst schwierig zu formulieren sind.

Um hier Abhilfe zu schaffen, wurde bereits eine Vielzahl von Polymeren vorgeschlagen, die an – meist statistisch verteilten – Seitenketten quartäre Ammoniumverbindungen aufweisen. So werden in den japanischen Patentanmeldungen 79 56 612 und 79 87 710 die Umsetzungsprodukte hydrolisierter Proteine mit Glycidyltrimethylammoniumchlorid vorgeschlagen. Die niederländische Patentanmeldung 76 12 362 nennt Polyacrylsäuren mit quartären Ammoniumseitengruppen. Mit Glycidyltrimethylammoniumchlorid umgesetzter Polyvinylalkohol ist Gegenstand der japanischen Anmeldung 77 03 689. Schliesslich sind zahlreiche Anmeldungen bekannt, die Saccharide, insbesondere Stärke oder Cellulose, mit quartären Ammoniumgruppen zum Gegenstand haben. Die genannten Polymere haben den Vorteil, dass sie wesentlich besser als die kationischen Tenside mit Aniontensiden verträglich sind und nicht zu Irritationen der Haut oder der Schleimhäute führen. Nachteilig ist jedoch die nach ihrer Anwendung beobachtete starke elektrostatische Aufladung des trockenen Haares, die in der Praxis bei Haarnachbehandlungsmitteln durch Zumischung von z. B. Cetyltrimethylammoniumchlorid ausgeglichen werden muss, wobei die vorgenannten Nachteile dieser Substanz in Kauf genommen werden müssen.

Es besteht somit ein Bedarf nach neuen quartären Ammoniumverbindungen, die den Vorteil polymerer Verbindungen mit den antistatischen Eigenschaften niedermolekularer Stoffe verbinden.

Aufgabe der Erfindung ist es somit, mehr funktionelle quartäre Ammoniumverbindungen mit Molekulargewicht 500–5000 und mit jeweils 1–2 quartären Ammoniumgruppen an beiden Kettenenden zu schaffen. Eine spezielle Aufgabe der Erfindung ist es, derartige Verbindungen aus leicht zugänglichen Ausgangsmaterialien aufzubauen, so durch Umsetzung von Oligomeren, insbesondere Polyethern, Polyestern oder Polyamiden, die an beiden Kettenenden Hydroxyl-, Carboxyl-, primäre Amino- oder sekundäre Aminogruppen aufweisen, mit Epoxiden, die eine quartäre Ammoniumgruppe enthalten.

Eine weitere Aufgabe der Erfindung besteht darin, ein Verfahren zur Herstellung der neuen quartären Ammoniumverbindungen mit je 1–2 quartären Ammoniumgruppen an beiden Kettenenden zu schaffen.

Weiterhin ist es Aufgabe der Erfindung, haarkosmetische Zubereitungen mit vorteilhaften Eigenschaften zu schaffen, welche die neuen quartären Ammoniumverbindungen enthalten.

Gegenstand der Erfindung ist somit in einer ersten Ausführungsform ein Verfahren zur Herstellung von mehrfunktionellen quartären Ammoniumverbindungen eines mittleren Molekulargewichtes zwischen 500 und 5000 durch Umsetzung von reaktiven Wasserstoff enthaltenden Polymerkomponenten mit quartären Ammoniumgruppen enthaltenden Epoxiden, dadurch gekennzeichnet, dass als reaktiven Wasserstoff enthaltende Polymerkomponenten unverzweigte Oligomere mit 1 oder 2 reaktiven H-Atomen an jedem Kettenende eingesetzt werden.

Ein weiterer Gegenstand der Erfindung sind die neuen mehrfunktionellen quartären Ammoniumverbindungen selbst.

In einer weiteren Ausführungsform ist Gegenstand der Erfindung die Verwendung der mehrfunktionellen quartären Ammoniumverbindungen in Haarpflege- oder Haarbehandlungsmitteln, insbesondere in Haarnachbehandlungsmitteln, Fönwellmitteln, Haarfestigern oder Haarsprays sowie Shampoos, Dauerwellmitteln oder Haarfärbemitteln.

Die Erfindung betrifft somit in einer ersten Ausführungsform Verbindungen, die an beiden Kettenenden je 1 oder 2 quartäre Ammoniumgruppen aufweisen. Die Gesamtfunktionalität an quartären Ammoniumgruppen kann somit bis zu 4 betragen. Die neuen mehrfunktionellen quartären Ammoniumverbindungen sind linear, d.h. sie weisen keine Seitenketten auf, die länger als die Hälfte der Hauptkette sind und insbesondere keine Seitenketten, welche ihrerseits quartäre Ammoniumgruppen tragen. Legt man diese Einschränkung zugrunde, so kann die Hauptkette jedoch in ihrer chemischen Natur, also z.B. Hydrophilie – Hydrophobie oder Kettenlänge in weiten Grenzen variiert werden.

Für haarkosmetische Zwecke besonders geeignete, erfindungsgemässe mehrfunktionelle quartäre Ammoniumverbindungen weisen ein Molekulargewicht zwischen 500 und 5000 auf, vorzugsweise zwischen 500 und 3000. Quartäre Ammoniumverbindungen mit höherem Molekulargewicht sind herstellbar, jedoch weisen sie – bezogen auf Gramm – nur einen recht geringen Anteil an quartären Ammoniumgruppen auf, so dass die gewünschten Effekte nur noch abgeschwächt zu beobachten sind.

In einer speziellen Ausführungsform betrifft die Erfindung die Umsetzungsprodukte von oligomeren Polyethern, die als reaktive Wasserstoffatome tragende Gruppen Hydroxyl-, Carboxyl- oder primäre oder sekundäre Aminogruppen an beiden Kettenenden aufweisen, mit quartären Ammoniumverbindungen, die eine Epoxidgruppe enthalten, insbesondere Glycidyltrialkyl($C_1$-$C_4$)-Halogenide. Hierbei wird die Umsetzung so geführt, dass jede Endgruppe des Oligomeren mindestens eine quartäre Ammoniumgruppe aufweist. Als Ausgangsmaterialien können zahlreiche bekannte und im Handel erhältliche Polyether verwendet werden. Geeignet sind beispielsweise die Kondensationsprodukte von Ethylenoxid, Propylenoxid und/oder Tetrahydrofuran. Diese weisen Hydroxylendgruppen auf. Geeignete Polyether mit Carboxylendgruppen sind beispielsweise Produkte, die aus den vorgenannten durch Veretherung mit Halogencarbonsäuren z. B. durch Veretherung mit Chloressigsäure herstellbar sind. Weitere geeignete Polyether mit Carboxylendgruppen sind aus Polyethern mit Hydroxylendgruppen durch Umsetzung mit je 1 Mol Dicarbonsäureanhydrid pro mol Hydroxylgruppe herstellbar. So sind die Umsetzungsprodukte von Polyethylenglykolen oder Polypropylenglykolen mit den Anhydriden der Bernsteinsäure, der Maleinsäure oder der Phthalsäure geeignet. Polyether mit Aminoendgruppen werden in zahlreichen Variationen angeboten. Dabei handelt es sich meist um Produkte auf Basis von Polyethylenglykol oder Polypropylenglykol oder deren Mischungen. Besonders geeignete Ausgangsmaterialien sind die unter dem Markennamen Jeffamin® im Handel angebotenen Produkte. Die Verwendung von aminogruppentragenden Polyethern als Ausgangsmaterial erlaubt es, höhermolekulare, quartäre Ammoniumverbindungen mit insgesamt bis zu 4 quartären Ammoniumgruppen pro Molekül herzustellen.

Die Umsetzung der Hydroxyl-, Carboxyl- oder aminoendgruppentragenden Polyether mit Glycidyltrialkylammoniumhalogeniden, insbesondere mit Glycidyltrimethylammoniumchlorid, erfolgt unter den für diese Reagenzien gängigen Bedingungen. So können hydroxylgruppenhaltige Materialien möglichst in Substanz oder in konzentrierten Lösungen bei Temperaturen von 100–140 °C, vorzugsweise unter basischer Katalyse umgesetzt werden. Carboxylgruppenhaltige Ausgangsmaterialien werden in Lösungsmitteln z. B. in Alkoholen oder Ketonen bei 80 °C und Reaktionszeiten bis zu 20 Stunden umgesetzt. Die Umsetzung von Oligomeren mit primären Aminoendgruppen hingegen erfolgt ebenfalls in alkoholischer Lösung bei Temperaturen um 30 °C oder darunter. Die Reaktionszeiten betragen hier vorzugsweise 2–5 Stunden.

Nach einer weiteren Ausführungsform betrifft die Erfindung höhermolekulare quartäre Ammoniumverbindungen aus Oligomeren, die durch Polykondensation bzw. Polyaddition entstanden sind. So können Polyester, Polyamide oder Polyurethane mit Hydroxyl-, Amino- oder Carboxylendgruppen als Ausgangsoligomere eingesetzt werden. Bei den durch Polykondensation hergestellten Oligomeren kann die gewünschte Art von Endgruppen durch Überschuss einer Komponente erzeugt werden. So können durch Kondensation von Dihydroxyverbindungen und Dicarbonsäuren Polyester mit endständigen OH-Gruppen hergestellt werden, indem man die Dihydroxyverbindung im Überschuss einsetzt.

Entsprechend entstehen Polyester mit Carboxylendgruppen bei Anwendung eines Überschusses an Dicarbonsäuren. Dies gilt sinngemäss auch für Polyamide sowie für Polyurethane, wobei hier endständige Isocyanatgruppen zu Aminogruppen verseift oder zu anderen reaktiven Gruppen umgewandelt werden können. Wird die Polykondensation oder Polyaddition bis zu vollständigem Umsatz geführt, so lässt sich über das Molverhältnis der Reaktanten auch das mittlere Molekulargewicht reproduzierbar einstellen. Hier gilt das allgemeine Fachwissen der Polymer-Chemie.

Polykondensationsprodukte sind auch aus Hydroxycarbonsäuren bzw. Aminocarbonsäuren oder deren reaktiven Derivaten, z. B. Siebenring-Lactonen oder Lactamen herstellbar, wobei zunächst Produkte mit unterschiedlichen Endgruppen entstehen. Durch Zugabe von Dicarbonsäuren lassen sich in diesem Fall Oligomere mit Carbonsäureendgruppen und definiertem mittlerem Molgewicht herstellen. Das Entsprechende gilt bei Zugabe von Diaminen. Auch hier sei auf Fachbücher der Polymer-Chemie, z. B. H. G. Elias, Makromoleküle, 4. Auflage, Hüthig & Wepf, Basel (1981), Seite 780 ff. und 796 ff., verwiesen.

Im einzelnen sind die folgenden durch Kondensation bzw. Polyaddition hergestellten Oligomeren mit Hydroxy-, Carboxyl- bzw. Aminoendgruppen besonders geeignete Ausgangsmaterialien zur Herstellung der erfindungsgemässen höhermolekularen quartären Ammoniumverbindungen.

1. Oligomere Polyester mit 2 Carboxylendgruppen, hergestellt durch Kondensation (Veresterung) von Dihydroxyverbindungen wie Ethylenglykol, Diethylenglykol, Triethylenglykol, Propylenglykol, Dipropylenglykol, oligomeren Propylenglykolen, Butandiol, Hexandiol einerseits mit einem Überschuss an aliphatischen geradkettigen Dicarbonsäuren mit 3–10 Kohlenstoffatomen, Phthalsäure und/oder deren Isomeren oder den reaktiven Derivaten dieser Verbindungen andererseits.

2. Oligomere Polyester mit Carboxylendgruppen, hergestellt durch Kondensation von Hydroxycarbonsäuren wie Glykolsäure, 3-Hydroxypropionsäure, Milchsäure, Äpfelsäure oder Weinsäure mit einem Unterschuss an aliphatischen Dicarbonsäuren mit 3–10 Kohlenstoffatomen.

3. Polyamide mit Carboxylendgruppen, hergestellt durch Kondensation von Diaminen, die 2–6 Kohlenstoffatome enthalten, mit einem Überschuss an aliphatischen Dicarbonsäuren, wie Malonsäure, Bernsteinsäure, Maleinsäure, Fumarsäure, Glutarsäure, Adipinsäure, Trimethyladipinsäure oder Sebacinsäure.

4. Polyamide mit Carboxylendgruppen, hergestellt durch Umsetzung von Caprolactam mit einem Unterschuss an $C_3$-$C_{10}$-Dicarbonsäuren.

5. Polyurethane mit Aminoendgruppen, hergestellt durch Polyaddition von Glykolen, wie Ethylenglykol, Propylenglykol, Butandiol, Hexandiol oder deren Kondensationsprodukten mit einem Überschuss an aliphatischen Diisocyanaten und anschliessender Verseifung restlicher Isocyanatgruppen zu Aminogruppen.

6. Polyamide mit Aminoendgruppen, hergestellt durch Umsetzung von Dicarbonsäuren, die 3–10 Kohlenstoffatome enthalten, mit einem Überschuss an Diaminen, die 2–10 Kohlenstoffatome enthalten, z. B. Ethylendiamin, Hexamethylendiamin oder Trimethylhexamethylendiamin.

7. Polyamide mit Aminoendgruppen, hergestellt durch Umsetzung von Caprolactam mit einem Unterschuss der vorstehend genannten Diamine.

Werden die genannten oligomeren Polykondensations- bzw. Polyadditionsprodukte mit Hydroxyl-, Carboxyl- oder Aminoendgruppen mit Glycidyltrialkylammoniumchlorid in die erfindungsgemässen Oligomeren mit 1–2 quartären Ammoniumgruppen je Kettenende überführt, so werden auch hier die bei der Umsetzung der Polyether-Oligomeren angegebenen Reaktionsbedingungen bevorzugt angewendet.

In einer bevorzugten Ausführungsform betrifft die Erfindung mehrfunktionelle quartäre Ammoniumverbindungen, hergestellt durch Umsetzung von Carboxylendgruppen aufweisenden Polymerisaten mit Epoxidgruppen enthaltenden quartären Ammoniumverbindungen. Bevorzugtes Ausgangsmaterial zur Herstellung dieser Produkte sind Polydiene mit Carboxylendgruppen, die in bekannter Weise durch Ozonolyse höhermolekularer Polydiene zugänglich sind. In gleicher Weise geeignet sind die Ozonolyse-Produkte von Copolymerisaten aus α-Olefinen, wie z. B. Propen mit Dienen wie z. B. Butadien. Zur Umsetzung der Carboxylgruppen tragenden Polymerisate zu den erfindungsgemässen höhermolekularen quartären Ammoniumverbindungen werden diese unter den beschriebenen Bedingungen mit Glycidyltrialkylammoniumchloriden, insbesondere mit Glycidyltrimethylammoniumchlorid umgesetzt.

Zur Herstellung erfindungsgemässer mehrfunktioneller quartärer Ammoniumverbindungen aus Oligomeren und epoxidgruppenhaltigen quartären Ammoniumverbindungen können ausser den Chloriden auch quartäre Ammoniumverbindungen mit anderen Gegenionen verwendet werden. Geeignet sind z. B. Bromide, Iodide, Fluoride, Methosulfate oder deren Mischungen. Weiterhin können die Alkylreste am quartären Stickstoffatom zwischen $C_1$-$C_4$ variiert werden.

Ein weiterer Gegenstand der Erfindung sind die höhermolekularen quartären Ammoniumverbindungen selbst. Diese weisen als Gruppe folgende Merkmale auf:

– an jedem Kettenende 1–2 quartäre Ammoniumgruppen, welche 3 Alkylreste der Kettenlänge $C_1$-$C_4$ aufweisen und deren Gegenion ein Halogenion OH- oder Methosulfat ist.

– einen $C_3$-Rest mit einer Hydroxylgruppe in 2-Stellung über den die quartäre Ammoniumgruppe an das endständige Sauerstoff- oder Stickstoffatom eines oligomeren Zwischengliedes gebunden ist.

– ein oligomeres Zwischenglied, das linear ist, d. h. das keine Seitengruppe trägt, die länger als die Hälfte der Kette ist oder ihrerseits eine quartäre Ammoniumgruppe aufweist.

Die oligomeren Zwischenglieder leiten sich von den Oligomeren ab, die vorstehend unter Punkt 1 bis Punkt 7 als geeignete Ausgangsmaterialien für die Herstellung der neuen, mehrfunktionellen quartären Ammoniumverbindungen beschrieben worden sind.

Ein weiterer Gegenstand der Erfindung ist die Verwendung der neuen, mehrfunktionellen quartären Ammoniumverbindungen in Haarpflege- oder Haarbehandlungsmitteln. Von den erfindungsgemässen Produkten sind hier insbesondere die Trimethylammoniumderivate geeignet. Diese gestatten die Formulierung hochwirksamer Nachbehandlungsmittel, wie z. B. Fönwellmittel oder Festiger, welche nicht nur elektrostatischen Aufladungen des trockenen Haares oder zu Haut- oder Schleimhautreizungen führen. Weiterhin heben sie beim Zusatz zu emulsionsförmigen Haarnachbehandlungsmitteln den Nachteil der verstärkten Haarbelastung nahezu vollständig auf. Die Einsatz-Konzentrationen der erfindungsgemässen Oligomeren in Haarnachbehandlungsmitteln liegen zwischen 0,1 und 10 Gew.-%, vorzugsweise zwischen 0,1 und 5 Gew.-%. Bei Fönwellmitteln sind 0,1 bis 5 Gew.-%, insbesondere 0,2 bis 3 Gew.-% bevorzugt.

Die erfindungsgemässen Oligomeren können weiterhin in Haarsprays eingesetzt werden. Sie zeigen dabei ausgeprägte antistatische Wirkung. Durch diese Anwendung, bei der feinverteilte Sprühnebel (Aerosole) entstehen, ist es besonders wichtig, dass die Oligomere höchstens geringfügig haut- oder schleimhautreizend sind. Geeignete Einsatzkonzentrationen in Haarsprays liegen zwischen 0,1 und 5 Gew.-%, vorzugsweise zwischen 0,2 und 3 Gew.-%.

Weiterhin können die erfindungsgemässen Oligomeren in Shampoos, Dauerwellmitteln oder Haarfärbemitteln eingesetzt werden. Auch bei dieser Anwendungsform wirken sie der elektrostatischen Aufladung des trockenen Haares entgegen. Ein weiterer Vorteil ist, dass keine Anreicherung auf dem Haar beobachtet wurde. Es tritt also keine Haarbelastung mit verschlechtertem Halt der Frisur auf. In jedem Falle ist das Haar im nassen Zustand gut kämmbar. Bei höheren Einsatzkonzentrationen des Polymeren, also über 3 Gew.-%, wurde zudem eine vielfach gewünschte leichte frisurfestigende Wirkung festgestellt.

Beispiele

1. Herstellung der erfindungsgemässen mehrfunktionellen quartären Ammoniumverbindungen.

A) Herstellung der Oligomeren
– allgemeine Vorschrift zur Herstellung von Oligoamiden mit endständigen Aminogruppen.

In einem Dreihalskolben mit Rührer und Destillationsaufsatz wird das Amin vorgelegt und die Dicarbonsäure zügig unter gelegentlicher Wasserkühlung zugegeben, wobei darauf geachtet werden muss, dass die Reaktionstemperatur zwischen 100 und 140 °C liegt, damit das Ammoniumsalz flüssig bleibt. Das Ammoniumalz wird unter Stickstoff im Verlauf von 6 Stunden langsam von 140 °C auf 200 °C hochgeheizt. Dabei spaltet sich bereits der grösste Anteil des Reaktionswassers, das den Umsatz der Amidbildung anzeigt, ab. Man lässt den Ansatz auf etwa 150 °C abkühlen, evakuiert vorsichtig auf 1333 Pa und vervollständigt die Amidbildung bei 200 °C und 1333 Pa. Das Produkt wird heiss abgefüllt. Die Zusammensetzung der Ansätze und die Oligomer- bzw. Polymereigenschaften sind der Tabelle I (A 4–A 9) zu entnehmen.

Tabelle I

Übersicht über die Oligomeren mit endständigen Aminogruppen

| Nr. | Edukte/mol | | Aminzahl | Molgewicht aus Aminzahl | Beschaffenheit |
|-----|-----------|---|----------|-------------------------|----------------|
| A 1 | Jeffamin D 230* | | 473 | 234 | viskos |
| A 2 | Jeffamin D 400* | | 279 | 362 | viskos |
| A 3 | Jeffamin D 2000* | | 54 | 2067 | viskos |
| A 4 | Adipinsäure, 1 | Trimethylhexamethylendiamin, 1,3 | 103 | 1089 | klar, fest |
| A 5 | Adipinsäure, 1 | Trimethylhexamethylendiamin, 1,1 | 39,6 | 2826 | klar, fest |
| A 6 | Bernsteinsäure, 1 | Trimethylhexamethylendiamin, 1,6 | 179 | 626 | klar, fest |
| A 7 | Bernsteinsäure, 1 | Trimethylhexamethylendiamin, 1,2 | 83 | 1344 | klar, fest, hellgelb |
| A 8 | Äpfelsäure, 1 | Trimethylhexamethylendiamin, 1,6 | 164 | 683 | fest, hellbraun |
| A 9 | Weinsäure, 1 | Trimethylhexamethylendiamin, 1,4 | 150 | 746 | fest, hellbraun |

* Polyether mit Aminoendgruppen der Firma Texaco (Warenzeichen)

– allgemeine Vorschrift zur Herstellung von Oligoestern mit endständigen Carboxylgruppen.

In einem Dreihalskolben mit Rührer und Destillationsbrücke werden Dicarbonsäure und Diol bzw. Hydroxycarbonsäure vorgelegt. Unter Stickstoff wird schnell auf 150 °C und dann im Verlauf von 6 Stunden von 150 auf 200 °C hochgeheizt. Dabei spaltet sich bereits der grösste Anteil des Reaktionswassers, das den Umsatz der Esterkondensation anzeigt, ab. Man lässt den Ansatz auf etwa 150 °C abkühlen, evakuiert vorsichtig auf 1333 Pa und vervollständigt den Umsatz bei 200 °C und 1333 Pa. Das Produkt wird heiss abgefüllt. Die Zusammensetzung der Ansätze und die Oligomereigenschaften sind der Tabelle II (A 10–A 15) zu entnehmen.

– allgemeine Vorschrift zur Herstellung von Oligoamiden mit endständigen Carboxylgruppen.

Die Herstellung erfolgt analog zu den Oligoamiden mit endständigen Aminogruppen. Die Zusammensetzung der Ansätze und die Eigenschaften der Oligomeren sind der Tabelle II (A 16–A 21) zu entnehmen.

Tabelle II

Übersicht über die dargestellten Oligomeren mit endständigen Carboxylgruppen

| Nr. | Edukte/mol | | Säurezahl | Molgewicht aus Aminzahl | Beschaffenheit |
|-----|-----------|---|-----------|-------------------------|----------------|
| A 10 | Adipinsäure, 1 | Glycolsäure, 1 | 620 | 181 | wachs, fest |
| A 11 | Adipinsäure, 1 | Glycolsäure, 2 | 456 | 246 | wachs, weich |
| A 12 | Adipinsäure, 1 | Glycolsäure, 4 | 333,8 | 336 | wachs, weich |
| A 13 | Adipinsäure, 1 | Glycolsäure, 8 | 224 | 500 | wachs, hart |
| A 14 | Adipinsäure, 2 | Hexamethylenglycol, 1 | 356 | 315 | wachs, hart |
| A 15 | Adipinsäure, 1,5 | Hexamethylenglycol, 1 | 185 | 606 | wachs, hart |
| A 16 | Adipinsäure, 2 | Trimethylhexamethylendiamin, 1 | 266 | 421 | sehr hochviskos |
| A 17 | Adipinsäure, 1,5 | Trimethylhexamethylendiamin, 1 | 167 | 672 | fest, hart |
| A 18 | Bascal[1], 2 | Hexamethylendiamin, 1 | 223 | 503 | hart, fest klebrig |
| A 19 | Bascal, 1,5 | Hexamethylendiamin, 1 | 129 | 871 | hart, spröde |
| A 20 | Trimethyladipinsäure, 2 | Trimethylhexamethylendiamin, 1 | 216 | 518 | fest, hart |
| A 21 | Trimethyladipinsäure, 1,75 | Trimethylhexamethylendiamin, 1 | 113 | 985 | fest, hart |

[1] Bascal, Dicarbonsäuregemisch der BASF (Warenzeichen)

B) Darstellung der quartären Ammoniumverbindungen (QAV)
aus aminogruppenterminierten Oligomeren

In einem Dreihalskolben mit Rührer, Rückflusskühler und Tropftrichter wird das aminogruppenterminisierte Oligomer als ethanolische Lösung vorgelegt. Unter Rühren und gelegentlicher Wasserkühlung wird vorsichtig eine konzentrierte ethanolische Lösung von Glycidyltrimethylammoniumchlorid (GMAC) so zugetropft, dass die Reaktionstemperatur 30 °C nicht übersteigt.

Pro primärer Aminogruppe wurden 2 Äquivalente GMAC eingesetzt. Im Anschluss wird 5 Stunden bei Raumtemperatur weitergerührt und das Lösungsmittel am Rotationsverdampfer abgezogen. Die Zusammensetzung der Ansätze und die Charakterisierung der QAV's sind der Tabelle III (B 1–B 9) zu entnehmen. Die QAV B 1 entsteht aus dem Oligomer A 1, QAV B 2 aus Oligomer A 2 usw.

Tabelle III
Übersicht über die dargestellten QAV's aus Oligomeren mit endständigen Aminogruppen

| Nr. | Epoxidzahl | Beschaffenheit |
|---|---|---|
| B 1 | 0,04 | fest, klebrig |
| B 2 | 0,14 | fest, klebrig |
| B 3, a* | 0,11 | fest, glasartig |
| B 3, b | 0,1 | hochviskos, hellgelb |
| B 4 | 0 | fest, hart |
| B 5 | 0,19 | fest, hart |
| B 6 | 0,05 | fest, klebrig |
| B 7 | 0 | fest, hart |
| B 8 | 0,18 | fest, hart |
| B 9 | 0,19 | fest, hart |

* pro primärer Aminogruppe wurde hier 1 Äquivalent Glycidyltrimethylammoniumchlorid eingesetzt.

C) Darstellung der quartären Ammoniumverbindungen (QAV's) aus carboxylgruppenterminierten Oligomeren

In einem Dreihalskolben mit Rührer, Rückflusskühler und Tropftrichter wird das carboxylgruppenterminierte Oligomer, gelöst in der gleichen Menge Ethanol, vorgelegt. Äquimolare Mengen GMAC werden zugegeben und bei 180 °C zur Reaktion gebracht. Der Umsatz wird anhand der Säurezahl verfolgt und die Reaktion abgebrochen, sobald die Säurezahl unter 20 abgesunken ist. Übliche Reaktionszeiten liegen bei 20–30 Stunden. Das Ethanol wird am Rotationsverdampfer abgezogen und das Produkt im Hochvakuum getrocknet. Die Zusammensetzung der Ansätze und die Charakterisierung der QAV's sind der Tabelle IV (B 10–B 21) zu entnehmen. Die QAV B 10 entsteht aus dem carboxylgruppenterminierten Oligomer A 10 usw.

Tabelle IV
Übersicht über die dargestellten quartären Ammoniumverbindungen (QAV's) aus Oligomeren mit endständigen Säuregruppen

| Nr. | Säurezahl | Epoxidzahl | Beschaffenheit |
|---|---|---|---|
| B 10 | 17 | 0 | hochviskos |
| B 11 | 31,5 | 0 | fest, teilkristallin |
| B 12 | 8 | 0 | wachsartig |
| B 13 | 7 | 0 | viskos |
| B 14 | 0,4 | 0 | wachsartig |
| B 15 | 7 | 0 | wachsartig |
| B 16 | 18 | 0 | fest, klebrig |
| B 17 | 9 | 0 | fest, hart |
| B 18 | 13 | 0 | fest, hart |
| B 19 | 15,2 | 0,1 | fest, hart |
| B 20 | 18 | 0 | fest, hart |
| B 21 | 9 | 0,14 | fest, klebrig |

2. Anwendung in haarkosmetischen Zubereitungen

1. Fettfreie Haarspülung
10,0 g Polymer B 14 und 12,0 g Hydroxyethylcellulose (Viskontran® HEC 30000 PR) wurden in 978 g Wasser gelöst.

2. Fönwelle
10,0 g Poly-N-vinylpyrrolidon, Molgewicht ca. 40000 (Luviskol® K 30) und 5,0 g quartäre Ammoniumverbindung B 20 wurden in 400 g Ethanol und 585 g Wasser gelöst. Beide Mittel verliehen dem Haar im nassen Zustand eine ausgezeichnete Kämmbarkeit und angenehme Weichheit. Im trokkenen Zustand war die elektrostatische Aufladung nahezu völlig verschwunden. Das Haar wies so gut wie keine Belastung auf und war ausgezeichnet frisierbar.

3. Shampoo mit antistatischer Wirkung
Es wurden die folgenden Bestandteile gemischt:
20,0 g  quartäre Ammoniumverbindung B 15
400,0 g  einer 25%igen wässrigen Lösung des Natriumsalzes von Laurylalkohol-2-EO-sulfat
100,0 g  einer 30%igen Lösung des N-Hydroxyethyl-N-Kokoalkylamidoethyl-carboxymethylglycinat-Na-Salzes
480,0 g  Wasser
7,5 g  Kochsalz

4. Haarfärbemittel
Es wurden gemischt:
80,0 g  Talgfettalkohol
250,0 g  25%ige wässrige Lösung von Natriumlaurylalkohol-2-EO-sulfat
30,0 g  quartäre Ammoniumverbindung B 9
15,0 g  p-Toluylendiamin
0,4 g  2,4-Diaminoanisol
2,3 g  p-Aminophenol
4,0 g  Resorcin
45,0 g  Ammoniak, 25%ig in Wasser
573,3 g  Wasser

Beide Mittel verliehen dem Haar im nassen Zustand eine gute Kämmbarkeit. Das Haar lud sich im trockenen Zustand nicht auf. Die Frisur erhielt Volumen und ausgezeichneten Halt.

## Patentansprüche

1. Verfahren zur Herstellung von mehrfunktionellen quartären Ammoniumverbindungen eines mittleren Molekulargewichtes zwischen 500 und 5000 durch Umsetzung von reaktiven Wasserstoff enthaltenden Polymerkomponenten mit quartären Ammoniumgruppen enthaltenden Epoxiden, dadurch gekennzeichnet, dass als reaktiven Wasserstoff enthaltende Polymerkomponenten unverzweigte Oligomere mit 1 oder 2 reaktiven H-Atomen an jedem Kettenende eingesetzt werden.

2. Verfahren nach Anspruch 1, dadurch gekennzeichnet, dass die unverzweigten Oligomeren an jedem Kettenende 1 oder 2 Carboxylgruppen, Hydroxylgruppen, primäre Aminogruppen oder sekundäre Aminogruppen aufweisen.

3. Verfahren nach den Ansprüchen 1–2, dadurch gekennzeichnet, dass die mehrfunktionellen quartären Ammoniumverbindungen ein mittleres Molekulargewicht (Zahlenmittel) zwischen 500 und 3000 aufweisen.

4. Verfahren nach den Ansprüchen 1–3, dadurch gekennzeichnet, dass man zur Herstellung der mehrfunktionellen quartären Ammoniumverbindungen von Oligoethern mit Hydroxyl- oder primären oder sekundären Aminoendgruppen ausgeht, insbesondere von Oligomeren des Ethylenoxids, Propylenoxids und/oder Tetrahydrofurans.

5. Verfahren nach den Ansprüchen 1–3, dadurch gekennzeichnet, dass man zur Herstellung der mehrfunktionellen quartären Ammoniumverbindungen von durch Polykondensation oder addierende Polykondensation (Polyaddition) hergestellten Oligomeren ausgeht, insbesondere von Polyurethanen, Polyamiden oder Polyestern mit Hydroxyl-, Carboxyl- oder Aminoendgruppen.

6. Verfahren nach Anspruch 5, dadurch gekennzeichnet, dass man zur Herstellung der höhermolekularen quartären Ammoniumverbindungen von mindestens einem der folgenden durch Polykondensation oder Polyaddition hergestellen Oligomeren ausgeht.

a) Kondensationsprodukte aus aliphatischen $C_3$-$C_{10}$-Dicarbonsäuren mit einem molaren Überschuss an $C_2$-$C_9$-Diamiden, insbesondere Ethylendiamin, Hexamethylendiamin oder Trimethylhexamethylendiamin,

b) Umsetzungsprodukte von aliphatischen $C_3$-$C_{10}$-Dicarbonsäuren mit Hydroxycarbonsäuren, insbesondere Glykolsäure,

c) Umsetzungsprodukte eines Überschusses von $C_3$-$C_{10}$-aliphatischen Dicarbonsäuren mit Glykolen, insbesondere Hexamethylenglykol,

d) Umsetzungsprodukte eines Überschusses an $C_3$-$C_{10}$-aliphatischen Dicarbonsäuren mit Diaminen, insbesondere Hexamethylendiamin oder Trimethylhexamethylendiamin.

7. Verfahren nach den Ansprüchen 1–3, dadurch gekennzeichnet, dass man zur Herstellung der mehrfunktionellen quartären Ammoniumverbindungen von durch radikalische Polymerisation hergestellten Polymerisaten mit Hydroxyl- oder Carboxylendgruppen, insbesondere von Polydienen mit Carboxylgruppen oder von carboxylgruppentragenden Copolymeren aus Dienen und α-Olefinen ausgeht.

8. Verfahren nach den Ansprüchen 1–7, dadurch gekennzeichnet, dass die quartäre Ammoniumgruppen enthaltenden Epoxide Glycidyltrialkylammoniumhalogenide sind, die pro Alkylkette nicht mehr als 4 C-Atome aufweisen und als Gegenion Chlor oder Brom enthalten.

9. Mehrfunktionelle quartäre Ammoniumverbindungen mit bis zu 4 quartären Ammoniumgruppen und einem Molekulargewicht von 500 bis 5000, hergestellt nach einem der Verfahren 1–8, dadurch gekennzeichnet, dass die quartären Ammoniumgruppen an den beiden Kettenenden eines oligomeren Zwischengliedes sitzen und jeweils über einen $C_3$-Rest, der in 2-Stellung eine OH-Gruppe aufweist, an Sauerstoff oder Stickstoffatome gebunden sind.

10. Mehrfunktionelle quartäre Ammoniumverbindungen nach Anspruch 9, dadurch gekennzeichnet, dass es sich bei den oligomeren Zwischengliedern um Polyether, Polyester, Polyamide, Polyolefine oder Polydiene handelt.

11. Verwendung der mehrfunktionellen quartären Ammoniumverbindungen, hergestellt nach den Ansprüchen 1–8, in Haarpflege- oder Haarbehandlungsmitteln, insbesondere in Haarnachbehandlungsmitteln, Fönwellmitteln, Haarfestigern oder Haarsprays sowie Shampoos, Dauerwellmitteln oder Haarfärbemitteln.

## Claims

1. A process for the production of polyfunctional quaternary ammonium compounds having an average molecular weight of from 500 to 5000 by reaction of polymer components containing reactive hydrogen with epoxides containing quaternary ammonium groups, characterized in that unbranched oligomers containing 1 or 2 reactive H-atoms at either end of the chain are used as the polymer components containing reactive hydrogen.

2. A process as claimed in claim 1, characterized in that the unbranched oligomers contain 1 or 2 carboxyl groups, hydroxyl groups, primary amino groups or secondary amino groups at either end of the chain.

3. A process as claimed in claims 1 and 2, characterized in that the polyfunctional quaternary ammonium compounds have an average molecular weight (number average) of from 500 to 3000.

4. A process as claimed in claims 1 to 3, characterized in that oligoethers containing hydroxyl or primary or secondary terminal amino groups, particularly oligomers of ethylene oxide, propylene oxide and/or tetrahydrofuran, are used as starting material for the production of the polyfunctional quaternary ammonium compounds.

5. A process as claimed in claims 1 to 3, characterized in that oligomers produced by polycondensation or adding polycondensation (polyaddition), particularly polyurethanes, polyamides or polyesters containing terminal hydroxyl, carboxyl or amino groups, are used as starting material for the production of the polyfunctional quaternary ammonium compounds.

6. A process as claimed in claim 5, characterized in that at least one of the following oligomers produced by polycondensation or polyaddition is used as starting material for the production of the relatively high molecular weight quaternary ammonium compounds:

a) condensation products of aliphatic $C_3$-$C_{10}$ dicarboxylic acids with a molar excess of $C_2$-$C_9$ diamines, particularly ethylene diamine, hexamethylene diamine or trimethyl hexamethylene diamine,

b) reaction products of aliphatic $C_3$-$C_{10}$ dicarboxylic acids with hydroxycarboxylic acids, particularly glycolic acid,

c) reaction products of an excess of $C_3$-$C_{10}$ aliphatic dicarboxylic acids with glycols, particularly hexamethylene glycol,

d) reaction products of an excess of $C_3$-$C_{10}$ aliphatic dicarboxylic acids with diamines, particularly hexamethylene diamine or trimethyl hexamethylene diamine.

7. A process as claimed in claims 1 to 3, characterized in that polymers containing terminal hydroxyl or carboxyl groups produced by radical polymerization, particularly polydienes containing carboxyl groups or diene/$\alpha$-olefin copolymers containing carboxyl groups, are used as starting material for the production of the polyfunctional quaternary ammonium compounds.

8. A process as claimed in claims 1 to 7, characterized in that the epoxides containing quaternary ammonium groups are glycidyl trialkylammonium halides which contain no more than 4 C-atoms per alkyl chain and which contain chlorine or bromine as counter-ion.

9. Polyfunctional quaternary ammonium compounds containing up to 4 quaternary ammonium groups and having a molecular weight of from 500 to 5000 produced by the process claimed in any of claims 1 to 8, characterized in that the quaternary ammonium groups are present at both chain ends of an oligomeric intermediate member and are each attached to oxygen or nitrogen atoms by a $C_3$-radical containing an OH-group in the 2-position.

10. Polyfunctional quaternary ammonium compounds as claimed in claim 9, characterized in that the oligomeric intermediate members are polyethers, polyesters, polyamides, polyolefins or polydienes.

11. The use of the polyfunctional quaternary ammonium compounds produced in accordance with claims 1 to 8 in hair care and hair treatment preparations, particularly in hair aftertreatment preparations, blow-wave preparations, hair lacquers or hair sprays and also shampoos, permanent wave preparations or hair dyes.

**Revendications**

1. Procédé pour la préparation de composés d'ammonium quaternaire polyfonctionnels ayant un poids moléculaire moyen compris entre 500 et 5000, par réaction de composants polymères contenant des atomes d'hydrogène réactifs, avec des époxydes contenant des groupes ammonium quaternaire, caractérisé en ce que l'on utilise en tant que composants polymères contenant des atomes d'hydrogène réactifs des oligomères non ramifiés ayant un ou deux atomes d'hydrogène réactifs à chaque bout de chaîne.

2. Procédé selon la revendication 1, caractérisé en ce que les oligomères non ramifiés présentent à chaque bout de chaîne un ou deux groupes carboxyle, groupes hydroxyle, groupes amino primaires ou groupes amino secondaires.

3. Procédé selon l'une quelconque des revendications 1–2, caractérisé en ce que les composés d'ammonium quaternaire polyfonctionnels présentent un poids moléculaire moyen (moyenne en nombre) compris entre 500 et 3000.

4. Procédé selon l'une quelconque des revendications 1 à 3, caractérisé en ce que, pour la préparation des composés d'ammonium quaternaire polyfonctionnels, on part d'oligomères comportant des groupes terminaux hydroxyle ou amino primaires ou secondaires, en particulier d'oligomères de l'oxyde d'éthylène, de l'oxyde de propylène et/ou du tétrahydrofuranne.

5. Procédé selon l'une quelconque des revendications 1 à 3, caractérisé en ce que, pour la préparation des composés d'ammonium quaternaire polyfonctionnels, on part d'oligomères obtenus par polycondensation ou polycondensation additive (poly-addition) en particulier de polyuréthannes, polyamides ou polyesters à groupes terminaux hydroxyle, carboxyle ou amino.

6. Procédé selon la revendication 5, caractérisé en ce que, pour la préparation des composés d'ammonium quaternaire à poids moléculaire élevé, on part d'au moins un des oligomères suivants, obtenus par polycondensation ou poly-addition:

a) des produits de condensation d'acides dicarboxyliques aliphatiques en $C_3$-$C_{10}$ avec un excès molaire de diamines en $C_2$-$C_9$, en particulier l'éthylènediamine, l'hexaméthylènediamine ou la triméthylhexaméthylènediamine;

b) des produits de réaction d'acides dicarboxyliques aliphatiques en $C_3$-$C_{10}$ avec des acides carboxyliques hydroxylés, en particulier l'acide glycolique;

c) des produits de réaction d'un excès d'acides dicarboxyliques aliphatiques en $C_3$-$C_{10}$ avec des glycols, en particulier l'hexaméthylèneglycol;

d) des produits de réaction d'un excès d'acides dicarboxyliques aliphatiques en $C_3$-$C_{10}$ avec des diamines, en particulier l'hexaméthylènediamine ou la triméthylhexaméthylènediamine.

7. Procédé selon l'une quelconque des revendications 1 à 3, caractérisé en ce que, pour la préparation des composés d'ammonium quaternaire polyfonctionnels, on part de polymères à groupes terminaux hydroxyle ou carboxyle, obtenus par polymérisation radicalaire, en particulier de polydiènes à groupes carboxyle, ou de copolymères, portant des groupes carboxyle, de diènes et d'α-oléfines.

8. Procédé selon l'une quelconque des revendication 1 à 7, caractérisé en ce que les époxydes contenant des groupes amino quaternaires sont des halogénures de glycidyltrialkylammonium, qui ne présentent pas plus de 4 atomes par chaîne alkyle et contiennent en tant que ion opposé du chlore ou du brome.

9. Composé d'ammonium quaternaire polyfonctionnels comportant jusqu'à 4 groupes ammonium quaternaire et ayant un poids moléculaire allant de 500 à 5000, préparés selon l'une quelconque des revendications 1 à 8, caractérisés en ce que les groupes ammonium quaternaire se trouvent aux deux extrémités de chaîne d'un chaînon intermédiaire oligomère et sont liés chaque fois à des atomes d'oxygène ou d'azote par l'intermédiaire d'un reste en C₃ qui présente un groupe OH en position 2.

10. Composé d'ammonium quaternaire polyfonctionnels selon la revendication 9, caractérisés en ce qu'il s'agit, pour les chaînons intermédiaires oligomères, de polyéthers, de polyesters, de polyamides, de polyoléfines ou de polydiènes.

11. Utilisation des composés d'ammonium quaternaire polyfonctionnels, préparés selon l'une quelconque des revendications 1 à 8, dans des produits de soins capillaires ou de traitement capillaire, en particulier dans des produits capillaires d'après traitement, des produits d'ondulation, des fixateurs ou des laques pour cheveux, ainsi que des shampooings, des produits pour permanentes ou des produits de teinture pour cheveux.